(19) Europäisches Patentamt
European Patent Office
Office européen des brevets

(11) EP 0 514 311 B2

(12) NEW EUROPEAN PATENT SPECIFICATION

(45) Date of publication and mention of the opposition decision:
**06.04.2005 Bulletin 2005/14**

(51) Int Cl.[7]: **A61L 2/18**, A01N 59/00, G02C 13/00, A61L 12/12

(45) Mention of the grant of the patent:
**18.01.1995 Bulletin 1995/03**

(21) Application number: **92500054.9**

(22) Date of filing: **06.05.1992**

# (54) Procedure for disinfecting and cleaning contact lenses

Verfahren zur Entkeimung und Reinigung von Kontaktlinsen

Procédé pour désinfecter et nettoyer les lentilles de contact

(84) Designated Contracting States:
**AT BE CH DE DK FR GB GR IT LI NL PT SE**

(30) Priority: **07.05.1991 ES 9101121**

(43) Date of publication of application:
**19.11.1992 Bulletin 1992/47**

(73) Proprietor: **DIRYGESA, S.L.**
**E-28009 Madrid (ES)**

(72) Inventors:
- **Nestares Padilla, Santiago**
  **E-28850 Torrejon de Ardoz (Madrid) (ES)**
- **Perales Rontome, Carlos**
  **E-28850 Torrejon de Ardoz (Madrid) (ES)**

(74) Representative:
**Bunke, Holger, Dr.rer.nat. Dipl.-Chem. et al**
**Prinz & Partner GbR**
**Manzingerweg 7**
**81241 München (DE)**

(56) References cited:
**EP-A- 0 082 798** **EP-A- 0 147 100**
**EP-A- 0 209 071** **EP-A- 0 219 220**
**EP-A- 0 426 489** **EP-A- 0 436 466**
**WO-A-86/05695** **WO-A-90/11786**
**WO-A-91/12825** **WO-A-91/12826**
**WO-A-91/19517** **WO-A-92/11041**
**WO-A-92/11042** **AU-B- 560 506**
**US-A- 4 473 550** **US-A- 4 568 517**
**US-A- 4 585 488**

- **WORLD PATENTS INDEX LATEST Week 9150, Derwent Publications Ltd., London, GB; AN91-363609**
- **F.Albert Cotton & Geoffrey Wilkinson, "Advanced Inorganic Chemistry", pages 414 to 416**

Remarks:
The file contains technical information submitted after the application was filed and not included in this specification

EP 0 514 311 B2

## Description

### SUBJECT OF INVENTION

The present invention refers to a process for disinfecting and cleaning contact lenses, comprising submerging them in a container containing a hydrogen peroxide solution of a concentration of between 0.5 and 6 % and catalase as a catalyst activating said hydrogen peroxide, to cause a controlled peroxide destabilization from the first instance and to potentiate its oxidating action in order to gain a more efficient lens disinfection and cleaning. As a final process outcome, the hydrogen peroxide degradation reaches levels allowing that the transformed solution be compatible with the eye and maintain the contact lens parameters.

### BACKGROUND OF INVENTION

Contact lenses in continuous use build up a series of fatty, proteinic or other kinds of deposits which act as substrates allowing environmental microorganisms being dangerous for the lens itself, and which may even be dangerous for the eye, deposit on them. Due to this, several means for disinfecting them have been devised, the most outstanding among them being those based on using oxygenated water which shows a high disinfecting power without damaging the lens morphology and leaving no noxious traces once it has been removed.

The use of oxygenated water, however, raises a problem just lying on the need to remove it once it has concluded its task, since any trace of this substance in a lens may cause serious irritations on the eye. Thus, the most conventional procedure consists in first disinfecting the lens with the oxygenated water contained in an adequate container and, after disinfection, to achieve in a second operation the neutralization of the oxygenated water by any known means (chemical compositions, catalytics, rinsing with saline water, etc.) in such a way that peroxide concentration in the solution may be reduced down to level not being damaging for eye.

Obviously, this two-phase method has the drawback of being slow and it even implies the risk that the user oversees adequate completion of the process.

In order to solve this inconvenience, other methods have been further on provided for, based in performing the disinfection and neutralization process in only one phase, that is, by introducing the lens in a container having simultaneously oxygenated water and all products required to neutralize it, forming at the same time an idoneous lens maintenance solution, with means to delay the neutralizing action by setting layers on it with appropriate substances which will dissolve themselves within some time, or other galenic preparation forms as described under Ciba-Geigy AG's patent 86/01791, some of them very difficult to perform.

This method, contrary to the one being proposed herein, does not seek to potentiate peroxide oxidative effect, but by delaying for some previously set time the start of the neutralizer action, it allows lenses to get in contact with peroxide at its maximum although stabilized concentration. It is after the above mentioned previously set time when the total neutralizer is released in a fast manner thus destroying hydrogen peroxide in order that lens causes no troubles to eye.

It is obvious that the only reason for including a neutralizer herein is to destroy hydrogen peroxide, seeking for no other effect.

Other methods for one-phase performance resource as well to neutralize oxygenated water by means of heavy metals catalysts, such as platinum, performing a slow neutralization that becomes lower the lesser is peroxide concentration.

To this system belongs the method protected by Gaglia's U.S.A. patent 3912451 of 10-14-75.

This method clearly differs from the one being proposed herein and seeks for different effects. According to it, contact lenses are submerged in normally stabilized oxygenated water in order to have them desinfected through its standard action. Simultaneously, or some time thereafter, a support provided with a metal type catalyst is submerged in the solution in order that it starts destroying oxygenated water by releasing oxygen, thus obtaining peroxide concentrations in the solution which are unable to affect the eye comfort once contact lens has been adapted to it.

There is no oxygenated water activation in this system. On the contrary, oxygen is only released at the metal catalyst surface, forming no complex at all, and this nascent oxygen is recombined to form an $O_2$ molecule whose oxidative power is very low. Therefore, peroxide solution is not being selectively activated (as it happens according to the present invention) in this method, but it is being disactivated. We may see, then, that this method only seeks the removal of oxygenated water from contact lens after disinfection, and that this removal is obtained by means of a metal catalyst.

Other single phase methods might exist which by using physical hindrances for releasing the neutralizer would delay oxygenated water degradation, but up to this time it has always been sought to obtain that peroxide concentration, stabilized always, be adequate during the period required for it to perform its disinfecting action.

Numerous patents describe methods for disinfecting contact lenses which use hydrogen peroxide as a disinfectant and the catalase enzyme to decompose hydrogen peroxide into water and oxygen, EP-A-0 209 071; WO-A-90 11 786.

The method being shown here aims for a different purpose. It is sought to improve hydrogen peroxide qualities in order that, when activating it, its action be faster and more efficient.

## DESCRIPTION OF THE INVENTION

The process of the present invention for cleaning and disinfecting contact lenses comprises submerging said lenses in a container containing a hydrogen peroxide solution of a concentration of between 0.5 and 6 % and catalase as a catalyst activating said hydrogen peroxide, wherein said catalase is introduced into said solution in the form of a tablet such that, as from the first moment, said catalase is continuously released from said tablet into said solution and that hydrogen peroxide degradation occurs down to a level allowing the solution to be compatible with the eye, with said lenses remaining in the solution between half an hour and two hours.

Preferred embodiments of the process according to the present invention additionally comprise the features of either one of pendant claims 2 to 9.

Hydrogen peroxide disinfecting action is based on oxidation taking place on the microorganism membranes until their destruction. This oxidation is performed by growing oxygen formed at peroxide decomposition:

$$H_2O_2 \rightarrow H_2O + O$$

Hydrogen peroxide is formed of two hydrogen atoms and two oxygen atoms. These atoms are linked through covalent bonds, one pair of electrons being shared by the hydrogen oxygen atoms; due to the water molecule configuration and the closer electrons'proximity to the oxygen nucleus, hydrogen becomes oxidated in a way and partially loses its electron, which is gained by oxygen. We may consider, therefore, that $H_2O_2$ molecule is ionized, constituted by an $O_2^{2-}$ anion and two $H^+$ cations. Reaction of metal peroxides, such as $Na_2O_2$ and $BaO_2$, with strong acids, such as hydrochloric and sulphuric, show that the $O_2^{2-}$ group (peroxide ion) may actually exist under ion quality.

Hydrogen peroxide may act as oxidant or reducer due to the fact that peroxide ion holds an intermediate place, so to say, among free molecular oxygen and oxide oxygen. The said ion may lose two electrons and become oxidated to free oxygen, or either gain them and become reduced to two oxide oxygens.

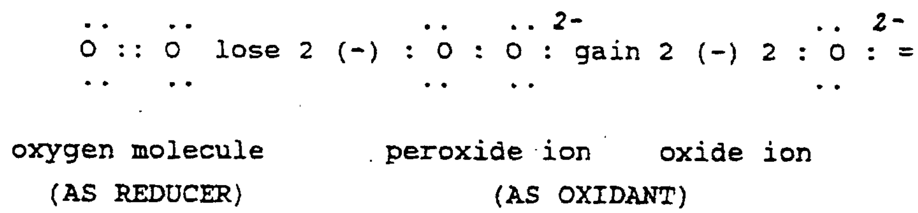


Due to this double oxide-reducer character it happens that an $H_2O_2$ molecule may act as oxidant on another molecule which would act as reducer, thus producing water and free oxygen.

$$2\ H_2O_2 \rightleftharpoons 2\ H_2O + O_2 + 46{,}200 \text{ calories}$$

Since molecules tend to place themselves at the lowest energy levels and being this an exothermal reaction, there is a tendency in this reaction to move right-hand wise and to the decomposition of hydrogen peroxide into water and molecular oxygen.

While this reaction takes place spontaneously in a slow manner, since it is affected by factors accelerating it such as light action, alkalies, dust particles, etc., marketed hydrogen peroxide solutions are usually stabilized with adequate additives and in an acid medium.

These stabilized solutions are those used for disinfecting. Although they have a remarkable germicide power, their oxidative reaction velocity in an acid medium is very low. Therefore, should we have available a method according to which, starting from a stabilized solution, we may obtain a given unstabilized level at time of using it, we would obtain an "activated" peroxide having much higher oxidating power.

If we introduce in oxygenated water a small amount of catalytic enzyme, catalase, a complex is formed between oxygenated water and the enzyme,

$$H_2O_2 + \text{enzyme} \rightarrow (H_2O_2 * \text{enzyme})$$

In this complex, oxygen lability linked with water is maximum, in such a way that should the solution have some unstabilizing factor (proteins, container physical irregularities, particles, etc.) these factors will suffice to split the complex and to free "in situ" nascent oxygen

$$(H_2O_2 * \text{enzyme}) + \text{factor} \rightarrow H_2O + \text{enzyme} + \text{factor - O}$$

A solution is thus obtained in which the complex, which may act at any point, is dissolved throughout its full mass.

It may happen, as well, the following reaction:

$$(H_2O_2 * \text{enzyme}) + (H_2O_2 * \text{enzyme}) \rightarrow 2\ H_2O + O_2 + 2\ \text{enzyme}$$

and all of it where activation factors lie. This will help us to visually check that it is on those points where oxygen is released that the highest oxidating action is taking place.

This may be physically seen in a clearly demonstrative manner, by comparison, peparing two oxygenated water solutions. First one (A) is a oxygenated water solution, 3 % for instance, stabilized with 0,1 % disodium EDTA, for example. The second (B) is the same (A) solution to which it has been added a sufficient amount of enzyme, for instance 400 U.K. catalase, to obtain the necessary unstabilizing and respective complex forming.

Should we submerge in each solution a glass strip on which some protein (for instance, egg denaturated albumin set with heat) has been placed, we may note that while on the one submerged in the A solution container no apparent phenomena take place, on the strip submerged in B solution, oxygen bubbles start forming immediately, above all on the surface wherein protein has been placed.

This is due to the immediate nascent oxygen attack as it is being released from the complex ($H_2O_2$ * enzyme) in the presence of protein in B solution. While in A solution, where no complex has been formed, the mere presence of protein is unable, at reasonably long term, to produce oxygen release. It is known that one of the products helping to decompose oxygenated water is protein, thus the higher easiness with which it is attacked by nascent oxygen released by peroxide decomposition and, therefore, since proteins are a part of the microorganisms' walls and other cellular structures thereof, these are destroyed in the presence of oxygenated water.

One more demonstration of the activation taking place in peroxide when it is unstabilized is the fast destruction of bacteria having catalase in this medium. That is due, as we have pointed out above, to the immediate formation of nascent oxygen taking place on these bacteria.

We should think that the lethal power of an "activated" hydrogen peroxide upon these microorganisms should be higher than that of a normally stabilized one. To ascertain it, we have chosen as test bench the action of both A and B solutions on a resistent microorganism in order to have a demonstrative test. We chose Candida utilis yeast with an implant of $2 \times 10^5$ microorganisms per ml of solution. Outcome is widely clarifying:

| Viable survivance | | |
|---|---|---|
| Time | A solution | B solution |
| 0 | $2 \times 10^5$ | $2 \times 10^5$ |
| 2 min | $9.5 \times 10^4$ | $4 \times 10^4$ |
| 5 min | $3 \times 10^4$ | $7 \times 10^3$ |
| 15 min | $1.8 \times 10^3$ | $4 \times 10^2$ |
| D (time required to lower viable concentration in one logarithmic unit) is 4.9 minutes for A solution and 1.8 minutes for B solution. | | |

It is clear that activated hydrogen peroxide has its germicide power increased and, as it may be seen in the table, its power during the acting times is 2.5 through 4.5 times higher.

"Activated" peroxide we are presenting in this invention has an icreased germicide power and is furthermore much more efficient for destroying dirt deposits built up on contact lenses surface. This is logical since activated hydrogen peroxide solution selectively releases nascent oxygen, in an immmediate manner, just at the dirty places, because it is there that the complex ($H_2O_2$ * enzyme) is destroyed. In this way, a high oxidative action takes place

exactly on the spot it is required, and it consequently destroys the dirt deposit structure, which is easily dissolved within the surrounding solution, just supported by the entraining circulation taking place at the oxygen release.

The following test has been performed:

100 glass strips measuring approximately 60 x 10 mm were submerged in an 0,2 % egg white albumin solution bath for 15 minutes. Once this time has elapsed, strips are moved to a dry heat stove at 140 °C leaving them there for 30 minutes to set protein through heat.

Protein coating formation was evaluated selecting 24 homogeneous strips.

Four of them were submerged in a saline solution (Fisiozor of Laboratorios Avizor). Ten in an A solution (3 % hydrogen peroxide solution stabilized with 0.1 % disodium EDTA). The remaining ten were submerged in a B solution (A solution with the addition of 400 U.K. catalase as unstabilizer).

Strips were taken off half an hour later, rinsed with saline solution and their cleanliness status was evaluated.

All ten samples treated with A solution showed a cleanliness status not higher than that of the four blank samples. Protein coating remained in both cases on 100 % of their surface.

Protein coating had disappeared from samples treated with B solution in a range between 70 % and 85 %.

Having available a hydrogen peroxide activated by oxygen lability implies a long step forward in contact lenses maintenance, since as lense caretaking operations are performed in relatively short periods of time, highly efficient products are required. Although stabilized peroxide has a noteworthy germicide power in short times, its acting on dirt deposits on the lense surface in the time lapses during which maintenance is performed by present methods is practically nil.

On the contrary, with the system we are preconizing, besides increasing germicice power, the action on dirt is enormously potentiated, so that, with activated peroxide, cleaning a lens within the short time span granted for the operation is really possible.

With this method we are clearly in advantage on those existing at the marketplace, since it has, on those requiring two phases, the advantages regarding use comfort we have discussed above besides avoiding dangerous mistakes. In regard with those comprising one single phase with metal catalysts, its advantage lies on performance time, safer neutralization and lower cost.

As regards other single-phase methods which could be marketed, it keeps all inherent goodnesses thereto we have already discussed. Besides, this method will allow lenses to be free of any chemical preservative. Resulting solution is free of them and they are not required since by performing this method germs are eliminated and, since it is performed in a closed container, lenses remain aseptic as long as we like, providing the container is not opened.

However, in any case, the reason why this method has its leading edge on all other methods is that it uses activated peroxide, which destroys germs much faster and, since it has labile oxygen available throughout the full process, it is able to fully obliterate microorganisms.

Where other peroxide methods reach scarcely significant cleaning levels, this one arrives at levels being high enough to usually make unnecessary the use of other cleaning products to keep lenses at an optimum wearing preservation status.

The method we are proposing consists in submerging the contact lens in a container wherein a $H_2O_2$ solution having a concentration of between 0.5 % and 6 %, has been poured. In order to reach aseptical action levels within short time lapses, preferably 10-15 minutes, 3 % peroxide concentrations are necessary. Also from the beginning, a peroxidase type catalytic enzyme, catalase, is introduced in solid form (tablet) in the solution, disposed in such a way as to be continuously, although slowly, released so that complex ($H_2O_2$ * enzyme) forming takes place in the solution to give rise to peroxide activation. It does not matter for how long lenses are submerged in the solution while process is taking place, although, due to user's comfort reasons, they remain in the solution between half an hour and two hours.

Continuous enzyme release may be graduated in order that within the selected time period an oxygen volume be released so that residual hydrogen peroxide concentration be lower than 50 ppm, for instance, a limit within which hydrogen peroxide causes no irritation on eye, although even full peroxide degradation may be achieved.

We may, thus, see that this process consists in submerging contact lenses in a hydrogen peroxide solution having adequate concentration, according with time lapse selected for asepticizing the lens, said peroxide being activated by unstabilization produced by gradual release, from the first moment, of a catalyst, so that throughout the time lapse selected, catalyst release be able to reduce hydrogen peroxide residual levels below the limits which could cause discomfort when contacting solution with eye.

Releasing catalyst in a controlled manner from the first moment may be performed in many ways.
As an example, without this implying any limitation whatsoever, we propose a tablet or bead carrying, together with the catalyst enzyme, other products to obtain a resulting solution having idoneous pH and osmolality to maintain the lenses parameters. These products may form buffers, such as boric acid and its salts, phosphates, etc. It may be used, as well, alkaline chlorides, disodium EDTA and other products.

Tablet or granule should be made in such a way as to have a catalyst concentration gradient being lower on its surface and higher in its nucleus. For this purpose the tablets may have several strata, or nucleated ones, or the

tablets may have an outer stratum with the catalyst held by slowly soluble products such as polyvinylpyrrolidones, polyacrylic acid, cellulose derivatives, etc., the catalyst in the nucleus being held by higher soluble products.

Or either the tablet, after having been made, is submitted to operations aiming to decrease the enzyme activity in a selective manner such as, for instance, with dry or wet heat or with products partially disactivating the catalyst, such as short chain alcohols.

Another solution could also be a tablet maintaining an acid pH on its surface which will make that its outer coating enzyme be partially destroyed, thus delaying the tablet dissolution. A tablet of this kind may be made by forming a nucleus wherein the enzyme is mixed with the coadjuvants maintaining the pH and the osmotic pressure, said nucleus being covered with innocuous acids such as tartaric or adipic acid.

Also a tablet not having a gradual action, but a phased one, may be made. To this end, a tablet, or any other galenic form, is made with three concentric strata. The outermost one holds an enzyme amount sufficient to obtain the formation of the complex activating peroxide. Middle stratum is neutral, with no enzyme, and will take for dissolving the time lapse chosen to make the complex act. Once this stratum is dissolved, the solution will get in contact with the innermost one, which carries the required catalyst to simultaneously continue forming the active complex and degrading peroxide up to level not being bothersome for the eye.

To make this tablet one may use, as outer stratum, a mixture of above mentioned products to obtain a correct final pH, or either soluble coatings integrating the catalyst.

Middle stratum may be formed out with products to cover the nucleus, which may be difficultly solving materials such as acrylic polymers, polyethyleneglycols, cellulose derivatives, polyvinylpyrrolidones, etc., or coatings of crystallizing products of small molecules as well, such as solid chloride or boric acid and its salts, etc.

Also a nucleated tablet may be made without resorting to coatings.

This method can be performed, for instance, by placing lenses within a container together with the hydrogen peroxide solution and a tablet having two concentric strata. It would have inside a peroxide neutralizer together with, besides other coadjuvant products, the acidic part of a buffer. Its outer area would carry at least the basic part of the buffer. Both strata may either be separated or not by a slow solubility coating in order to delay acting of the peroxide neutralizer down to required levels.

This method has a much higher germicide and cleaning power than that of other methods based on delayed tablets which only seek for posterior acting of the neutralizer.

We have mentioned here some ways to carry out this method, the invention being not limited to them.

## PREFERRED EMBODIMENT OF THE INVENTION

The process to disinfect and clean contact lenses is fundamentally carried out by means of two substances. On one side, a hydrogen peroxide solution, preferably at 0,5 % to 3 % weight, and, on the other, a catalyst being capable to form an active complex with peroxide, namely catalase. This catalyst may be contained in a tablet, either as sole component thereof or together with other complementary substances to complete the lens maintenance process.

As such, the disinfecting and cleaning process consists in submerging the lenses in a container having, at the same time, a hydrogen peroxide solution and a tablet containing the enzymatic catalyst placed in a manner that will allow to the gradual release of same, thus activating the peroxide, which will disinfect and clean the lens. When the tablet has fully dissolved, the amount of enzymatic catalyst released will be such that it may degrade, during the established time lapse, the whole peroxide present in the solution, which will have been transformed due to the addition of the other components in a regulated pH and osmolality solution similar to tears, which will maintain the lens parameters and will be compatible with the eye.

Besides, user may keep the lens stored in its closed container for as long as he/she likes.

Process has been disposed to end within the term of half an hour to two hours, although different time lapses may be chosen.

For performing this method a tablet may be preferably made having a nucleus and a coating. The nucleus would carry the catalase, being sufficient to determine the peroxide degradation and it may carry as well a part of the other raw materials used for obtaining the pH and osmolality sought for. These raw materials may be boric acid and sodium tetraborate, monosodium and disodium phosphates, sodium chloride, potassium chloride, disodium EDTA and other excipients. Coating would carry enough catalase to unstabilize peroxide and form an active complex, and it may also carry in its composition matters like those it carries in its nucleus, such as sodium chloride and boric acid, and it may carry, as well, other components making its solubility slower, such as polymers of the polyvinylpyrrolidone, polyacrylic acid, polyethyleneglycols, etc., types.

## Claims

1. A process for cleaning and disinfecting contact lenses comprising submerging said lenses in a container containing a hydrogen peroxide solution of a concentration of between 0.5 and 6% and catalase as a catalyst activating said hydrogen peroxide, **characterized in that** said catalase is introduced into said solution in the form of a tablet such that, as from the first moment, said catalase is continuously released from said tablet into said solution and that hydrogen peroxide degradation occurs down to a level allowing the solution to be compatible with the eye, with said lenses remaining in the solution between half an hour and two hours.

2. The process of claim 1, wherein the acting time of said catalase constitutes the full lense disinfecting time or the largest part thereof.

3. The process of claim 1, wherein said catalase is distributed in the tablet mass in a uniform manner.

4. The process of claim 1, wherein said catalase is distributed in the tablet mass in a non-uniform manner with a concentration gradient which makes it increase towards the interior of said tablet.

5. The process of claim 1, wherein said hydrogen peroxide level allowing the solution to be compatible with the eye is not higher than 50 ppm.

6. The process of claim 1, wherein said tablet contains a salt concentration constituting a buffer to stabilize the solution pH.

7. The process of claim 6, wherein said solution pH is between 6 and 8.

8. The process of claim 7, wherein said solution pH is between 6.5 and 7.5.

9. The process of claim 1, wherein the components of said tablet confer to said solution a tonicity similar to that one of tear liquid.

## Patentansprüche

1. Verfahren zum Reinigen und Desinfizieren von Kontaktlinsen, bei dem die Linsen in einen Behälter eingetaucht werden, der eine Wasserstoffperoxidlösung einer Konzentration von zwischen 0,5 und 6 % und Katalase als Katalysator zur Aktivierung des Wasserstoffperoxids enthält, **dadurch gekennzeichnet, daß** die Katalase in Form einer Tablette in die Lösung eingebracht wird, so daß die Katalase vom ersten Augenblick an kontinuierlich aus der Tablette in die Lösung freigesetzt wird und das Wasserstoffperoxid bis zu einer Konzentration hinunter abgebaut wird, bei der die Lösung für das Auge verträglich ist, wobei die Linsen während eines Zeitraums zwischen einer halben Stunde und zwei Stunden in der Lösung verbleiben.

2. Verfahren nach Anspruch 1, bei dem die Einwirkzeit der Katalase die Gesamtdesinfektionszeit der Linse oder den größten Teil davon darstellt.

3. Verfahren nach Anspruch 1, bei dem die Katalase gleichmäßig in der Tablettenmasse verteilt ist.

4. Verfahren nach Anspruch 1, bei dem die Katalase ungleichmäßig in der Tablettenmasse mit einem Konzentrationsgradienten verteilt ist, der die Konzentration in Richtung auf das Innere der Tablette ansteigen läßt.

5. Verfahren nach Anspruch 1, bei dem die Wasserstoffperoxidkonzentration, bei der die Lösung für das Auge verträglich ist, nicht höher als 50 ppm ist.

6. Verfahren nach Anspruch 1, bei dem die Tablette eine einen Puffer bildende Salzkonzentration zur Stabilisierung des pH-Werts der Lösung enthält.

7. Verfahren nach Anspruch 6, bei dem der pH-Wert der Lösung zwischen 6 und 8 liegt.

8. Verfahren nach Anspruch 7, bei dem der pH-Wert der Lösung zwischen 6,5 und 7,5 liegt.

**9.** Verfahren nach Anspruch 1, bei dem die Bestandteile der Tablette der Lösung eine der Tränenflüssigkeit ähnliche Tonizität verleihen.

## Revendications

**1.** Procédé pour nettoyer et désinfecter des lentilles de contact, dans lequel les lentilles sont plongées dans un récipient qui contient une solution de peroxyde d'hydrogène ayant une concentration entre 0,5 et 6 % et de la catalase servant de catalyseur pour activer le peroxyde d'hydrogène, **caractérisé en ce que** la catalase est introduite dans la solution sous forme de comprimé, de telle sorte que la catalase est libérée à partir du comprimé dans la solution dès le premier instant de manière continue et que le peroxyde d'hydrogène est décomposé jusqu'à une concentration à laquelle la solution est bien tolérée par l'oeil, les lentilles restant dans la solution pendant un laps de temps entre une demi-heure et deux heures.

**2.** Procédé selon la revendication 1, dans lequel le temps d'action de la catalase constitue le temps de désinfection total de la lentille ou la grande partie de celui-ci.

**3.** Procédé selon la revendication 1, dans lequel la catalase est répartie de façon régulière dans la masse du comprimé.

**4.** Procédé selon la revendication 1, dans lequel la catalase est répartie de façon irrégulière dans la masse du comprimé avec un gradient de concentration qui fait monter la concentration en direction d'intérieur du comprimé.

**5.** Procédé selon la revendication 1, dans lequel la concentration de peroxyde d'hydrogène, à laquelle la solution est bien tolérée par l'oeil, n'est pas supérieure à 50 ppm.

**6.** Procédé selon la revendication 1, dans lequel le comprimé contient une concentration de sel formant tampon pour stabiliser la valeur de pH de la solution.

**7.** Procédé selon la revendication 6, dans lequel la valeur de pH de la solution est de 6 à 8.

**8.** Procédé selon la revendication 7, dans lequel la valeur de pH de la solution est de 6,5 à 7,5.

**9.** Procédé selon la revendication 1, dans lequel les composants du comprimé confèrent à la solution une tonicité qui est semblable au liquide lacrymal.